# EUROPEAN PATENT SPECIFICATION

(11) **EP 3 159 043 B1**
(45) Date of publication and mention of the grant of the patent: **20.11.2019**
(21) Application number: 16179350.0
(22) Date of filing: 13.07.2016
(51) Int. Cl.: A61Q 1/10, A61K 8/34, A61K 8/35, A61K 8/362

(54) **PEEL-OFF TYPE EYEBROW COLORING COMPOSITION AND MANUFACTURING METHOD THEREOF**
ABSCHÄLBARE AUGENBRAUENFÄRBUNGSZUSAMMENSETZUNG UND HERSTELLUNGSVERFAHREN DAFÜR
COMPOSITION DE COLORATION DU SOURCIL DE TYPE PEEL-OFF ET SON PROCÉDÉ DE FABRICATION

(30) Priority: 23.10.2015 KR 20150147971
(43) Date of publication of application: 26.04.2017
(73) Proprietor: C&C International Co., Ltd., Suwon-si, Gyeonggi-do (KR)
(72) Inventor: BAE, Eun-chul, Yongin-si Gyeonggi-do (KR)
(74) Representative: Ruzzu, Giammario

(56) References cited:
- US-B1- 6 458 390
- DATABASE GNPD [Online] MINTEL; 1 June 2016 (2016-06-01), Anonymous: "Tint My Brows Gel", XP002766125, Database accession no. 4092389

## Description

### BACKGROUND OF THE INVENTION

The present invention relates to a peel-off type eyebrow coloring composition and its manufacturing method, and more particularly to a peel-off type eyebrow coloring composition and its manufacturing method, which composition is excellent in tinting power and color staying power and easy to use and also has an effect to exfoliate dead cells from eyebrows.

The shape, size, position and color of eyebrows are the factors that greatly determine the impression of a person, so across all the ages and countries of the world, there have been sustained attempts to maintain the shape and color of the eyebrows in beauty.

But, the eyebrows are a part of the biological tissue consisting of hair, and the location, luxuriant growth and thickness of eyebrow hair vary depending on the body features of each person. In particular, eyebrow hair loses and gets thinner with aging. This makes it very difficult to maintain eyebrows in beauty and good shape.

It is also quite cumbersome to groom eyebrows with a razor and daily tint eyebrows in a desired color with an eyebrow tint that is a cosmetic preparation available for single use. For that reason, some people remove all the eyebrow hair and get eyebrow tattoo in a desired shape and color to semi-permanently maintain their eyebrows in good shape. But, such an eyebrow make-up with tattoo ends up not properly coping with the person's changing beauty taste and beauty trends.

The following cited patent document 1 discloses an eyebrow-shaped sponge that realizes an eyebrow make-up by stamping rather than by drawing. But, this technique involves too short retention time of the eyebrow make-up and somewhat inconvenience of application.

The following cited patent document 2 describes an eyebrow pencil composition that uses an oil-soluble film-forming agent in addition to a conventional eyebrow pencil composition and contains a moisturizer and a water-soluble color emulsified by an emulsifying agent to enhance the retention of the make-up. But, this technique is just providing a temporary tint to the surface of the skin, so it involves poor tinting power and poor color staying power. Further, it fails to guarantee an eyebrow tinting in a desired color with satisfaction, to form an elastic coating film, or to create a look of fuller, thicker brows.

Accordingly, there is an urgent demand for an eyebrow coloring composition that penetrates deep into the skin as well as on the surface of the skin to provide good tinting power, forms an elastic coating film in the eyebrow region, causes no skin irritation, dries fast, offers good color staying power, and retains high quality of color light for a long time, thereby creating a natural look of eyebrow.

### [Prior Art Patent Documents]

Patent Document 1: KR Utility Patent Publication No. 20-2011-0005915 (06/15/2011)
Patent Document 2: KR Patent Publication No. 10-1996-000193 (01/25/1996).

Article "Tint My Brows Gel", GNPD, MINTEL, (20160601), Database accession no. 4092389, XP002766125 [IP] 1-5 * Section: "Ingredients" refers to an eyebrow pencil composition that uses an oil-soluble film-forming agent in addition to a conventional eyebrow makeup base product to enhance the retention of the make-up. The product thick eyebrows which can be shaped as desired, and requires a minimum dry for two hours. After that it is completely dried, it can be removed carefully from behind the eyebrows.

This article was published between the priority date and the filing date of the present patent application, and therefore it is not relevant prior art for the claimed subject matter.

Document US6458390 disclosed an invention in the field of compositions for application to eyebrows and eyelashes to impart color, or for the use in lining the eyes with color, or application to the skin as in the facial or body tattoos or the lips as a semi-permanent lipcolor. In particular the invention of US6458390 relates to a single phase gel based makeup composition containing one or more iron oxide pigments, a film forming silicone acrylate copolymer solvated in volatile solvent, and one or more non-wax gelling agents. The gelling agents are capable of gelling the composition to a viscosity of at least about 1,000 centipoise at room temperature. Also disclosed is the use of such composition, either alone or when emulsified into a polar phase.

### BRIEF SUMMARY OF THE INVENTION

For solving the above-mentioned problems with the prior art, the inventors of the present invention have contrived the present invention based on the discovery that a novel peel-off type eyebrow coloring composition can be used to provide an eyebrow coloring composition that penetrates deep into the skin as well as on the surface of the skin to provide good tinting power, forms an elastic coating film in the eyebrow region, causes no skin irritation, dries fast, and offers good color staying power, thereby retaining high quality of color light for a long time.

It is therefore an object of the present invention to provide a peel-off type eyebrow coloring composition capable of providing good tinting power and good color staying power.

It is another object of the present invention to provide a method for manufacturing an eyebrow coloring composition that provides good tinting power and good color staying power.

In order to achieve the one object of the present invention, in accordance with one embodiment of the present invention, there is provided a peel-off type eyebrow coloring composition that includes 15 to 30 parts by weight of water, 0.01 to 0.05 part by weight of disodium EDTA, 0.5 to 2 parts by weight of polysorbate, 6 to 12 parts by weight of polyvinyl alcohol, 2 to 15 parts by weight of polyvinyl pyrrolidone, 0.3 to 6 parts by weight of a color, 1 to 30 parts by weight of dihydroxy acetone, 0.1 to 0.5 part by weight of phenoxy ethanol, 0.1 to 0.5 part by weight of citric acid, and 5 to 30 parts by weight of ethyl alcohol.

In accordance with another embodiment of the present invention, the peel-off type eyebrow coloring composition may further include 10 to 20 parts by weight of butylene glycol.

In accordance with further another embodiment of the present invention, the peel-off type eyebrow coloring composition may further include 1 to 5 parts by weight of 1,2-hexanediol.

In accordance with another embodiment of the present invention, the peel-off type eyebrow coloring composition may further include a fragrance.

In order to achieve the other object of the present invention, there is provided a method for manufacturing a peel-off type eyebrow coloring composition that includes: (a) dissolving 0.01 to 0.05 part by weight of disodium EDTA and 0.5 to 2 parts by weight of polysorbate in 15 to 30 parts by weight of water at 75 to 85 °C to prepare a first solution; (b) gradually adding 6 to 12 parts by weight of polyvinyl alcohol to the first solution under agitation and performing a complete dissolution to prepare a second solution; (c) gradually adding 2 to 15 parts by weight of polyvinyl pyrrolidone to the second solution under agitation and completing a dispersion to prepare a third solution; (d) adding 0.3 to 6 parts by weight of a color and 1 to 30 parts by weight of dihydroxy acetone to the third solution under agitation and completing a dispersion to prepare a fourth solution; (e) adding 0.1 to 0.5 part by weight of phenoxy ethanol and 0.1 to 0.5 part by weight of citric acid to the fourth solution under agitation and completing a dispersion to prepare a fifth solution; and (f) adding 5 to 30 parts by weight of ethyl alcohol to the fifth solution under agitation and completing a dispersion to prepare a sixth solution.

### EFFECTS OF THE INVENTION

The peel-off type eyebrow coloring composition according to the present invention penetrates deep into the skin as well as on the surface of the skin to provide good tinting power, forms an elastic coating film in the eyebrow region, causes no skin irritation, dries very fast to provide convenience of use, offers good color staying power, causing no change of the initial color with an elapse of time, and retains the shape of eyebrow with high quality of color light for a long time. In addition, the eyebrow make-up using the peel-off type eyebrow coloring composition creates a natural look of eyebrow almost without being washed away or smudged with water or sweat. Further, the composition of the present invention has a merit to exfoliate in the eyebrow region.

### BRIEF DESCRIPTION OF THE SEVERAL VIEW OF THE DRAWING

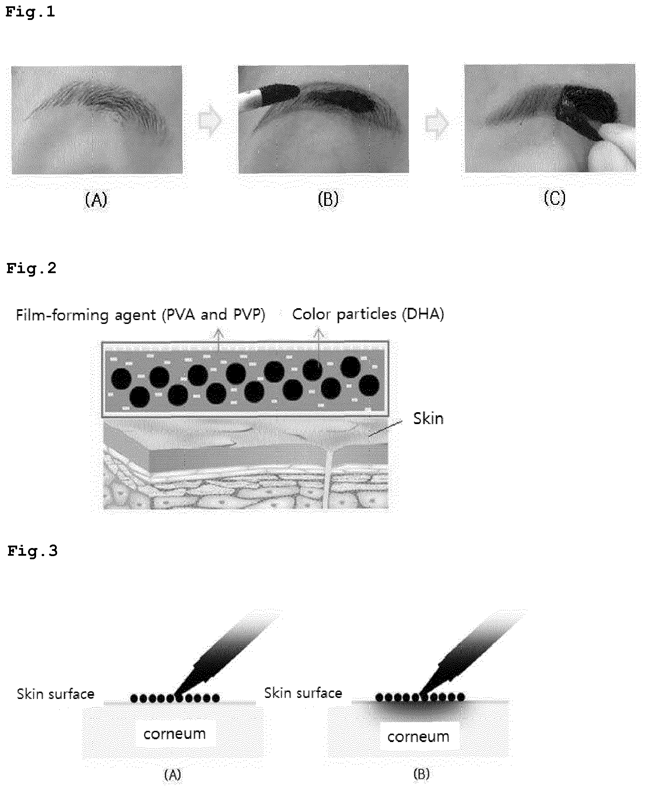

FIG. 1 is an illustrative image showing the process of tinting an eyebrow with the peel-off type eyebrow coloring composition of the present invention.
FIG. 2 is an illustration showing the functions of polyvinyl alcohol (PVA), polyvinyl pyrrolidone (PVP) and dihydroxy acetone (DHA) contained in the peel-off type eyebrow coloring composition of the present invention.
FIG. 3 is an illustration showing the good tinting power of the peel-off type eyebrow coloring composition of the present invention.

### DETAILED DESCRIPTION OF THE INVENTION

Prior to the further specific description of the present invention, it should be understood that the terms used in this disclosure and the claims are not to be confined to the common or dictionary meanings but interpreted to have meanings and concepts coinciding with the technical conceptions of the present invention on the basis of the principle that the concepts of the terms can be properly defined for the sake of the best explanation of the present invention. Therefore, specific details disclosed herein are not to be interpreted as representing all the technical conceptions of the present invention but given as a preferred example of the present invention. Obviously, many equivalents and variations that may replace the embodiments given herein are possible in the light of the teaching of the present disclosure.

Hereinafter, the present invention will be described in further detail with reference to the preferred embodiments in order for those skilled in the art to embody the present invention with ease. In the following description of the present invention, a detailed description of known functions and configurations incorporated herein will be omitted when it may make the subject matter of the present invention rather unclear.

The present invention relates to a peel-off type eyebrow coloring composition and its manufacturing method, and more particularly to a peel-off type eyebrow coloring composition and its manufacturing method, which composition is excellent in tinting power and color staying power and easy to use and also has an effect to exfoliate dead cells from eyebrows.

The eyebrow coloring composition according to one embodiment of the present invention is an advanced concept tattoo type eyebrow make-up using a double liquid crystal coating method to form double films, so it can make a long-lasting tinting effect that the eyebrow tint lasts for about 3 to 5 days with no more than a single application.

The double liquid crystal coating method employed in the present invention uses an ionic dye and a volatile polymer that cause no skin irritation. According to this coating method, a desired color can be rapidly absorbed by the skin of the eyebrow region and stuck firmly in the skin.

Also, the eyebrow coloring composition of the present invention is of a peel-off type and thus very easy and simple to use.

The eyebrow coloring composition of the present invention basically includes a solvent, a film-forming agent, a color, a coloring reinforcing agent, a preservative, and a pH adjuster.

Examples of the solvent as used herein may include water, butylene glycol, or ethyl alcohol.

Preferably, water is distilled water or deionized water and contained in an amount of 15 to 30 parts by weight. When the water content is less than 15 parts by weight, a lack of the water-soluble solvent leads to poor solubility of the different functional components and hence deterioration in the skin feeling after use. When the water content is greater than 30 parts by weight, the content of the different functional components is too low in relation to water, so it cannot give a satisfactory effect such as good tinting power with an appropriate amount of the composition applied to the skin.

Butylene glycol may be used as an auxiliary solvent for water. If used, butylene glycol is contained in an amount of 10 to 20 parts by weight. When the content of butylene glycol is less than 10 parts by weight, the effect of the auxiliary solvent is insignificant. When the content of butylene glycol is greater than 20 parts by weight, the amount of water used as the main solvent is so relatively small as to deteriorate the entire feeling after use of the composition.

Ethyl alcohol is the last solvent added to the composition of the present invention and contained in an amount of 5 to 30 parts by weight. When the content of ethylene alcohol is less than 5 parts by weight, the volatilization rate of the solvent is so low as to possibly reduce the drying rate of the composition. When the content of ethylene alcohol is greater than 30 parts by weight, the volatilization rate of the solvent is so high that the composition dries out before the color completely penetrates into the skin to complete a tinting.

In regards to the contents of the solvents included in the composition of the present invention, it is therefore preferable to use 15 to 3 parts by weight of water and 5 to 30 parts by weight of ethyl alcohol; or 15 to 30 parts by weight of water, 10 to 20 parts by weight of butylene glycol and 5 to 30 parts by weight of ethyl alcohol.

Water and butylene glycol may be used together as a mixed solvent, and ethyl alcohol is added separately apart from the water or butylene glycol.

Examples of the film-forming agent as used herein are polyvinyl alcohol (PVA) and polyvinyl pyrrolidone (PVP). Particularly, the composition of the present invention is characterized by using a combination of polyvinyl alcohol and polyvinyl pyrrolidone as film-forming agents.

These film-forming agents have both a function of forming the composition of the present invention into a film available as a peel-off type film and a function of coating the remaining tint portion of the eyebrow region after the composition is peeled off to protect the tint portion against water or sweat and thereby to retain the tinting power for a long time.

Polyvinyl alcohol, which has a film-forming function, is used in facial masks and also available as an emulsification stabilizer. Polyvinyl alcohol has a relatively short drying time, high adhesive strength and a function to form a strong coating film. Polyvinyl alcohol is commonly used as a principal matrix polymer of a general hydrogel patch and has non-toxicity, non-carcinogenicity, biocompatibility, high mechanical properties, easy processability, and high water uptake.

Polyvinyl pyrrolidone exhibits both a film-forming function and good adherence to hair and is thus used in hair care preparations and also in shampoo in order to stabilize bubbles and to make hair glossy. A small amount of polyvinyl pyrrolidone added in the cosmetics such as eyebrow tints forms a film as thin as invisible. Polyvinyl pyrrolidone is also used as an irritation reducer in the preparations that tend to cause skin irritations upon long-term contact with the skin.

The composition of the present invention comprises 6 to 12 parts by weight of polyvinyl alcohol and 2 to 15 parts by weight of polyvinyl pyrrolidone. When the content of polyvinyl alcohol is less than 6 parts by weight or that of polyvinyl pyrrolidone is less than 2 parts by weight, the composition of the present invention applied to the eyebrows dries out and is not easy to peel off, and the tint portion remaining in the eyebrow deteriorates in regards to the coating-forming function after removal of the composition and is thus unable to remain stably for a long time upon exposure to water or sweat. On the other hand, when the content of polyvinyl alcohol is greater than 12 parts by weight or that of polyvinyl pyrrolidone is greater than 15 parts by weight, the composition of the present invention has too high viscosity to be easily applied to the eyebrow region, and the content of other functional substances, such as the color, dihydroxy acetone, phenoxy ethanol, and citric acid, is relatively low to cause deterioration of the tinting power, decomposition of the composition after a long-term storage and discoloration of the composition due to the failure to maintain an appropriate pH.

The color may include any color in different colors as selected according to the preference of the user. Particularly, for the sake of creating a natural look of eyebrow color, the color as used herein may be Blue No. 1 (Red 1, CI 42090), Red No. 227 (Red 33, CI 17200), food color Yellow No. 5 (Yellow 6, CI 15985), or a combination of these colors. The color is contained in the composition of the present invention in an amount of 0.3 to 6 parts by weight. When the content of the color is less than 0.3 part by weight, the composition has an insignificant tinting effect. When the content of the color is greater than 6 parts by weight, it is uneconomical to use the color more than enough to give a tinting effect.

The coloring reinforcing agent serves to help the color penetrate deep into the eyebrow skin. Examples of the coloring reinforcing agent as used herein include dihydroxy acetone (DHA).

Dihydroxy acetone is contained in the composition of the present invention in an amount of 1 to 30 parts by weight. When the content of dihydroxy acetone is less than 1 part by weight, it deteriorates the effect of reinforcing the penetration of the color into the skin. When the content of dihydroxy acetone is greater than 30 parts by weight, the relative content of the solvent and the film-forming agent is so low as to deteriorate the feeling after use of the composition and the peel-off efficiency of the composition. Even if the given amount of the color or dihydroxy acetone is used, the final tinting effect on the skin may vary depending on the user's personal skin constitution.

The eyebrow coloring composition of the present invention also includes a preservative and a pH adjuster. 0.1 to 0.5 part by weight of phenoxy ethanol is used as the preservative, and citric acid is used as the pH adjuster. When the content of phenoxy ethanol is less than 0.1 part by weight, the composition of the present invention possibly spoils in the case of long-term storage. When the content of phenoxy ethanol is greater than 0.5 part by weight, there occurs an unnecessary reaction with the film-forming agent or the like to cause a risk of hardening the composition. When the content of citric acid is less than 0.1 part by weight, the composition may discolor after application to the eyebrow. When the content of citric acid is greater than 0.5 part by weight, the pH value of the composition gets lower to make the composition acidic.

The composition of the present invention may further include disodium EDTA that deactivates a trace of metal ions possibly entering the cosmetic composition through bonding with them. The disodium EDTA is contained in the composition in an amount of 0.01 to 0.05 part by weight. When the content of disodium EDTA is less than 0.01 part by weight, a trance of metal ions remaining in the composition of the present invention can not be kept from making a catalytic function in the skin, leading to the failure to maximize the stability of the composition and causing deterioration of the feeling after use or discoloration of the tinting portion. When the content of disodium EDTA is greater than 0.05 part by weight, it is uneconomical.

The composition of the present invention may further include polysorbate as a solubilizer that serves to help the above-mentioned functional components uniformly dissolve in the solvent. The polysorbate as used herein is polysorbate 80 and used in an amount of 0.5 to 2 parts by weight. When the content of polysorbate is less than 0.2 part by weight, the functional components contained in the composition of the present invention cannot uniformly dissolve in the solvent. When the content of polysorbate is greater than 2 parts by weight, it is uneconomical.

The composition of the present invention may further include 1,2-hexanediol. The 1,2-hexanediol is contained in the composition in an amount of 1 to 5 parts by weight. When the content of 1,2-hexandiol is less than 1 part by weight, it has an insignificant effect of moisturization and preservation. When the content of 1,2-hexanediol is greater than 5 parts by weight, there is a risk of causing a potential toxicity in the composition.

The composition of the present invention may further include a fragrance for providing a scent.

Hereinafter, a detailed description will be given as to the method for manufacturing a peel-off type eyebrow coloring composition according to the present invention.

Firstly, 0.01 to 0.05 part by weight of disodium EDTA and 0.5 to 2 parts by weight of polysorbate are dissolved in 15 to 30 parts by weight of water at 75 to 85 °C, for example, 80 °C to prepare a first solution. In this regard, the water, disodium EDTA and polysorbate are added into a clean stainless pot or the like.

Subsequently, while the first solution is kept at 75 to 85 °C, for example, 80 °C and under agitation with an Agi-mixer or the like for 30 to 40 minutes, 6 to 12 parts by weight of polyvinyl alcohol is gradually added to the first solution and completely dissolved to prepare a second solution.

Subsequently, while the second solution is kept at 75 to 85 °C, for example, 80 °C and under agitation with an Agi-mixer or the like for 10 to 20 minutes, 2 to 15 parts by weight of polyvinyl pyrrolidone is gradually added to the second solution and completely dispersed to prepare a third solution.

Subsequently, with the third solution under agitation with an Agi-mixer or the like, 0.3 to 6 parts by weight of a color and 1 to 30 parts by weight of dihydroxy acetone are added to the third solution and completely dispersed to prepare a fourth solution.

Subsequently, with the fourth solution under agitation with an Agi-mixer or the like, 0.1 to 0.5 part by weight of phenoxy ethanol and 0.1 to 0.5 part by weight of citric acid are added to the fourth solution and completely dispersed to prepare a fifth solution.

Subsequently, with the fifth solution under agitation with an Agi-mixer or the like, 5 to 30 parts by weight of ethyl alcohol is added to the fifth solution and dispersed to prepare a sixth solution. This process of preparing the sixth solution is carried out at the room temperature of 15 to 25 °C, and the sixth solution is the eyebrow coloring composition of the present invention.

In the step of obtaining the first solution, 10 to 20 parts by weight of butylene glycol may be further added.

In the step of obtaining the fifth solution, 1 to 5 parts by weight of 1,2-hexanediol may be further added.

In the step of obtaining the sixth solution, 0.01 to 2 parts by weight of the fragrance may be further added.

In every step of the preparation process for the composition of the present invention, the solution is subjected to visual evaluation in regards to dispersion and color to determine whether a sufficiently uniform dissolution is completed.

Additionally, the sixth solution is filtered through a filter of about 100 mesh to eliminate foreign substances possibly contained in the composition.

When necessary, about 10 wt.% of extra water may be added to the sixth solution.

The sixth solution thus obtained is sealed off so that no air or no foreign substance can get it.

Hereinafter, the present invention will be described in further detail through the following examples and comparative examples, which are not given to limit the scope of the present invention.

### Example 1

20.26 g of distilled water, 0.03 g of disodium EDTA, 0.5 g of polysorbate 80 (RHEODOL TW-O120V), and 10 g of butylene glycol (1,3 G/G) are added into a washed stainless pot and dissolved at 80 °C for about 20 minutes. While the port is kept at about 80 °C under agitation with an Agi-mixer, 9 g of polyvinyl alcohol (PVA-217) is gradually added and completely dissolved for about 35 minutes. Under continuous agitation with the Agi-mixer, 11 g of polyvinyl pyrrolidone (K-30) is gradually added and completely dispersed (80 °C). Then, 0.12 g of Blue No. 1, 0.14 g of Red No. 227, 1.15 g of Yellow No. 5, and 25 g of dihydroxy acetone are added and uniformly dispersed with the Agi-mixer. Under continuous agitation with the Agi-mixer, 2 g of 1,2-hexanediol, 0.3 g of phenoxy ethanol and 0.2 g of citric acid (F6000) are added, and 20 g of ethyl alcohol and 0.3 g of greengrass fragrance (HP-317235) are added at the room temperature. The final product thus obtained is filtered through a 100-mesh filter to prepare a peel-off type eyebrow coloring composition of the present invention, which is sealed off for storage.

### Comparative Example 1: Exclusion of Dihydroxy Acetone

20.26 g of distilled water, 0.03 g of disodium EDTA, 0.5 g of polysorbate 80 (RHEODOL TW-O120V), and 10 g of butylene glycol (1,3 G/G) are added into a washed stainless pot and dissolved at 80 °C for about 20 minutes. While the port is kept at about 80 °C under agitation with an Agi-mixer, 9 g of polyvinyl alcohol (PVA-217) is gradually added and completely dissolved for about 35 minutes. Under continuous agitation with the Agi-mixer, 11 g of polyvinyl pyrrolidone (K-30) is gradually added and completely dispersed (80 °C). Then, 0.12 g of Blue No. 1, 0.14 g of Red No. 227, and 1.15 g of Yellow No. 5 are added and uniformly dispersed with the Agi-mixer. Under continuous agitation with the Agi-mixer, 2 g of 1,2-hexanediol, 0.3 g of phenoxy ethanol and 0.2 g of citric acid (F6000) are added, and 20 g of ethyl alcohol and 0.3 g of greengrass fragrance (HP-317235) are added at the room temperature. The final product thus obtained is filtered through a 100-mesh filter to prepare a peel-off type eyebrow coloring composition of the present invention, which is sealed off for storage.

### Comparative Example 2: Exclusion of Polyvinyl Pyrrolidone

20.26 g of distilled water, 0.03 g of disodium EDTA, 0.5 g of polysorbate 80 (RHEODOL TW-O120V), and 10 g of butylene glycol (1,3 G/G) are added into a washed stainless pot and dissolved at 80 °C for about 20 minutes. While the port is kept at about 80 °C under agitation with an Agi-mixer, 20 g of polyvinyl alcohol (PVA-217) is gradually added and completely dissolved for about 35 minutes. Then, 0.12 g of Blue No. 1, 0.14 g of Red No. 227, 1.15 g of Yellow No. 5, and 25 g of dihydroxy acetone are added and uniformly dispersed with the Agi-mixer. Under continuous agitation with the Agi-mixer, 2 g of 1,2-hexanediol, 0.3 g of phenoxy ethanol and 0.2 g of citric acid (F6000) are added, and 20 g of ethyl alcohol and 0.3 g of greengrass fragrance (HP-317235) are added at the room temperature. The final product thus obtained is filtered through a 100-mesh filter to prepare a peel-off type eyebrow coloring composition of the present invention, which is sealed off for storage.

### Comparative Example 3: Exclusion of Polyvinyl Alcohol

20.26 g of distilled water, 0.03 g of disodium EDTA, 0.5 g of polysorbate 80 (RHEODOL TW-O120V), and 10 g of butylene glycol (1,3 G/G) are added into a washed stainless pot and dissolved at 80 °C for about 20 minutes. Under continuous agitation with the Agi-mixer, 11 g of polyvinyl pyrrolidone (K-30) is gradually added and completely dispersed (80 °C). Then, 0.12 g of Blue No. 1, 0.14 g of Red No. 227, 1.15 g of Yellow No. 5, and 25 g of dihydroxy acetone are added and uniformly dispersed with the Agi-mixer. Under continuous agitation with the Agi-mixer, 2 g of 1,2-hexanediol, 0.3 g of phenoxy ethanol and 0.2 g of citric acid (F6000) are added, and 20 g of ethyl alcohol and 0.3 g of greengrass fragrance (HP-317235) are added at the room temperature. The final product thus obtained is filtered through a 100-mesh filter to prepare a peel-off type eyebrow coloring composition of the present invention, which is sealed off for storage.

The eyebrow coloring compositions prepared in the Example 1 and the Comparative Examples 1, 2 and 3 are subjected to the following performance testings.

The following testings are conducted on 10 females (specimens 1 to 10) 21 to 30 years old and wearing no facial make-up and 10 females (specimens 11 to 20) 31 to 40 years old and wearing no facial make-up. All the test females wash their faces with warm water using a face cleanser once, and in 30 minutes after the cleaning, they apply the given composition of the present invention onto their eyebrow region to a thickness of about 0.5 to 1. Each test female conducts the application and removal testing using the compositions of Example 1 and Comparative Examples 1, 2 and 3 and tint her eyebrows with the compositions of Example 1 and Comparative Examples 1, 2 and 3 at five-day intervals, with a different composition sample applied to each eyebrow. Hence, it takes 10 days in total to conduct all the testings. Before the testings, no information about the composition samples, that is, which samples correspond to Example 1 or Comparative Examples 1-3 is given to the test females. Each test female is allowed to decide the application order of the composition samples so that each composition sample can be applied to the testings in random order. In about 60 minutes after application of the composition sample, each test female is told to remove (peel off) the composition sample. In this regard, the composition sample is peeled off in a same direction (that is, pulled toward the center of the face).

### [Tinting power Testing]

The compositions of Example 1 and Comparative Examples 1, 2 and 3 are used to tint the eyebrows, and the degree of coloring is visually evaluated and rated on a scale of 1 to 5 as follows:
1: Very good
2: Good
3: Fair
2: Poor
1: Very poor (no tinting)

The test results are presented in Tables 1 to 4.

### [Peel-Off Performance Testing]

The compositions of Example 1 and Comparative Examples 1, 2 and 3 are applied to eyebrows, and in about 60 minutes, peeled off in the above-defined direction with a hand. The residual of the compositions on the eyebrows after the peel-off is visually evaluated and rated on a scale of 1 to 5 as follows:
5: No residual
4: Completely peeled off with a little residual.
3: Peeled off with one try and a little residual.
2: Peeled off with more than one try and a lot of residual.
1: Nearly failure to peel off.

The test results are presented in Tables 1 to 4.

### [Feeling Testing - Sensory Test]

20 test subjects who apply the compositions of Example 1 and Comparative Examples 1, 2 and 3 to their eyebrows are subjected to a sensory test in regards to the feelings after use. The feelings after use are categorized into the feeling during application, the feeling during peel-off, and the feeling on the eyebrows during five-day use. These feelings are entirely evaluated and rated on a scale of 1 to 5 as follows:
5: Very good
4: Good
3: Fair
2: Poor
1: Very poor

The test results are presented in Tables 1 to 4.

### [Color staying power Testing]

The compositions of Example 1 and Comparative Examples 1, 2 and 3 are used to tint the eyebrows. A test is conducted to determined how long the initial color lasts in one day, three days, and five days after the application of the compositions. For a better comparison of the eyebrow tinting, the eyebrow regions of each test participant are photographed immediately, one day and three days after the application of the compositions. The photographs are used to determine the retention of the color in the eyebrow regions. The color staying power is evaluated according to the change of the color and rated on a scale of 1 to 5 as follows:
5: No color change observed.
4: Nearly no change of color, no need of additional tinting, and the still-lasting eyebrow outline.
3: Color and eyebrow outline fade slightly.
2: Color and eyebrow outline fade a lot.
1: Looks like before the application, and the color is gone.

The test results are presented in Tables 1 to 4.

**[Table 1]**

| Example | Specimen | Tinting power | Peel-off performance | Feeling after use | Color staying power | | |
|---|---|---|---|---|---|---|---|
| | | | | | 1 day | 3 day | 5 day |
| 1 | 1 | 5 | 4 | 5 | 5 | 5 | 4 |
| 1 | 2 | 5 | 5 | 5 | 5 | 5 | 4 |
| 1 | 3 | 5 | 5 | 5 | 5 | 5 | 5 |
| 1 | 4 | 5 | 5 | 4 | 5 | 4 | 4 |
| 1 | 5 | 5 | 4 | 5 | 5 | 4 | 4 |
| 1 | 6 | 5 | 4 | 5 | 5 | 5 | 4 |
| 1 | 7 | 4 | 5 | 4 | 5 | 5 | 5 |
| 1 | 8 | 5 | 5 | 4 | 5 | 5 | 4 |
| 1 | 9 | 5 | 4 | 5 | 5 | 4 | 4 |
| 1 | 10 | 5 | 4 | 5 | 5 | 4 | 4 |
| 1 | 11 | 4 | 5 | 4 | 5 | 5 | 5 |
| 1 | 12 | 5 | 5 | 4 | 5 | 5 | 5 |
| 1 | 13 | 5 | 5 | 5 | 5 | 5 | 5 |
| 1 | 14 | 5 | 4 | 5 | 5 | 4 | 4 |
| 1 | 15 | 4 | 5 | 5 | 5 | 4 | 4 |
| 1 | 16 | 5 | 5 | 5 | 5 | 5 | 5 |
| 1 | 17 | 5 | 5 | 5 | 5 | 5 | 5 |
| 1 | 18 | 5 | 5 | 5 | 5 | 5 | 5 |
| 1 | 19 | 5 | 5 | 5 | 5 | 4 | 4 |
| 1 | 20 | 5 | 4 | 4 | 5 | 4 | 4 |

**[Table 2]**

| Comparative Example | Specimen | Tinting power | Peel-off performance | Feeling after use | Color staying power | | |
|---|---|---|---|---|---|---|---|
| | | | | | 1 day | 3 day | 5 day |
| 1 | 1 | 3 | 4 | 5 | 4 | 1 | 1 |
| 1 | 2 | 3 | 5 | 4 | 4 | 2 | 1 |
| 1 | 3 | 2 | 5 | 5 | 4 | 1 | 1 |
| 1 | 4 | 3 | 5 | 5 | 4 | 2 | 1 |
| 1 | 5 | 3 | 4 | 4 | 4 | 2 | 1 |
| 1 | 6 | 2 | 4 | 5 | 5 | 2 | 1 |
| 1 | 7 | 3 | 5 | 4 | 4 | 2 | 1 |
| 1 | 8 | 2 | 5 | 5 | 4 | 2 | 1 |
| 1 | 9 | 2 | 5 | 5 | 4 | 1 | 1 |
| 1 | 10 | 3 | 4 | 5 | 4 | 2 | 1 |
| 1 | 11 | 2 | 5 | 4 | 4 | 1 | 1 |
| 1 | 12 | 3 | 5 | 5 | 5 | 2 | 1 |
| 1 | 13 | 2 | 4 | 4 | 4 | 1 | 1 |
| 1 | 14 | 2 | 5 | 5 | 4 | 2 | 1 |
| 1 | 15 | 3 | 5 | 4 | 4 | 2 | 1 |
| 1 | 16 | 2 | 5 | 5 | 4 | 1 | 1 |
| 1 | 17 | 3 | 4 | 4 | 4 | 2 | 1 |
| 1 | 18 | 2 | 5 | 5 | 4 | 1 | 1 |
| 1 | 19 | 3 | 4 | 4 | 4 | 2 | 1 |
| 1 | 20 | 2 | 5 | 5 | 4 | 1 | 1 |

**[Table 3]**

| Comparative Example | Specimen | Tinting power | Peel-off performance | Feeling after use | Color staying power | | |
|---|---|---|---|---|---|---|---|
| | | | | | 1 day | 3 day | 5 day |
| 2 | 1 | 4 | 2 | 3 | 4 | 3 | 3 |
| 2 | 2 | 4 | 2 | 4 | 4 | 3 | 2 |
| 2 | 3 | 5 | 2 | 3 | 4 | 3 | 3 |
| 2 | 4 | 4 | 3 | 3 | 5 | 3 | 2 |
| 2 | 5 | 5 | 2 | 3 | 5 | 2 | 2 |
| 2 | 6 | 4 | 2 | 4 | 5 | 3 | 3 |
| 2 | 7 | 4 | 3 | 3 | 4 | 3 | 2 |
| 2 | 8 | 5 | 2 | 3 | 5 | 3 | 3 |
| 2 | 9 | 4 | 3 | 3 | 4 | 4 | 2 |
| 2 | 10 | 4 | 2 | 3 | 5 | 2 | 2 |
| 2 | 11 | 4 | 2 | 4 | 5 | 3 | 2 |
| 2 | 12 | 5 | 3 | 3 | 5 | 2 | 2 |
| 2 | 13 | 4 | 2 | 3 | 4 | 3 | 2 |
| 2 | 14 | 5 | 3 | 3 | 5 | 2 | 3 |
| 2 | 15 | 4 | 3 | 4 | 4 | 3 | 2 |
| 2 | 16 | 4 | 3 | 3 | 5 | 3 | 2 |
| 2 | 17 | 4 | 2 | 3 | 4 | 3 | 2 |
| 2 | 18 | 5 | 3 | 3 | 5 | 2 | 2 |
| 2 | 19 | 4 | 2 | 4 | 4 | 3 | 2 |
| 2 | 20 | 4 | 3 | 3 | 4 | 3 | 2 |

**[Table 4]**

| Comparative Example | Specimen | Tinting power | Peel-off performance | Feeling after use | Color staying power | | |
|---|---|---|---|---|---|---|---|
| | | | | | 1 day | 3 day | 5 day |
| 3 | 1 | 4 | 3 | 3 | 3 | 3 | 2 |
| 3 | 2 | 4 | 2 | 4 | 3 | 3 | 1 |
| 3 | 3 | 4 | 3 | 3 | 4 | 3 | 2 |
| 3 | 4 | 5 | 2 | 3 | 3 | 3 | 1 |
| 3 | 5 | 4 | 3 | 3 | 4 | 3 | 2 |
| 3 | 6 | 5 | 3 | 4 | 4 | 3 | 2 |
| 3 | 7 | 4 | 2 | 3 | 4 | 2 | 2 |
| 3 | 8 | 4 | 3 | 3 | 4 | 3 | 2 |
| 3 | 9 | 4 | 2 | 3 | 3 | 3 | 2 |
| 3 | 10 | 4 | 3 | 3 | 4 | 3 | 2 |
| 3 | 11 | 5 | 2 | 3 | 3 | 2 | 2 |
| 3 | 12 | 4 | 3 | 2 | 3 | 2 | 1 |
| 3 | 13 | 5 | 3 | 3 | 3 | 2 | 2 |
| 3 | 14 | 4 | 3 | 3 | 4 | 3 | 1 |
| 3 | 15 | 4 | 3 | 3 | 4 | 2 | 2 |
| 3 | 16 | 4 | 3 | 3 | 3 | 3 | 1 |
| 3 | 17 | 4 | 3 | 2 | 3 | 3 | 2 |
| 3 | 18 | 4 | 3 | 3 | 4 | 2 | 1 |
| 3 | 19 | 4 | 2 | 2 | 3 | 2 | 2 |
| 3 | 20 | 4 | 3 | 3 | 4 | 3 | 2 |

As can be seen from the results of Tables 1 to 4, Example 1 that corresponds to the composition of the present invention is relatively very good in the tinting power in comparison with Comparative Example 1 and in the peel-off performance, feeling after use and color staying power in comparison with Comparative Examples 1 and 2.

### [Skin Toxicity Testing]

The composition of the present invention prepared in Example 1 is evaluated in regards to the skin toxicity in the testing conducted on 20 test participants. The skin toxicity of the composition is determined by (1) having the participants record in the test paper whether there is itching in the eyebrow region during the test; (2) having the test participants peel off the composition and wash their face to observe any sign of skin irritation such as rash in the eyebrow region in 5 days after the completion of the color staying power testing. The test results are presented in Table 5.

**[Table 5]**

| Skin Toxicity Test | | | | |
|---|---|---|---|---|
| Example | Specimen | Test items | | |
| | | Itching | Rash | Other skin irritations |
| 1 | 1 | No | No | No |
| 1 | 2 | No | No | No |
| 1 | 3 | No | No | No |
| 1 | 4 | No | No | No |
| 1 | 5 | No | No | No |
| 1 | 6 | No | No | No |
| 1 | 7 | No | No | No |
| 1 | 8 | No | No | No |
| 1 | 9 | No | No | No |
| 1 | 10 | No | No | No |
| 1 | 11 | No | No | No |
| 1 | 12 | No | No | No |
| 1 | 13 | No | No | No |
| 1 | 14 | No | No | No |
| 1 | 15 | No | No | No |
| 1 | 16 | No | No | No |
| 1 | 17 | No | No | No |
| 1 | 18 | No | No | No |
| 1 | 19 | No | No | No |
| 1 | 20 | No | No | No |

As can be seen from Table 5, the eyebrow coloring composition of the present invention causes no skin irritation including itching, rash, or the like to all the test participants. These results are construed to back up the stability of the eyebrow coloring composition of the present invention.

## Claims

1. A peel-off type eyebrow coloring composition comprising 15 to 30 parts by weight of water, 0.01 to 0.05 part by weight of disodium EDTA, 0.5 to 2 parts by weight of polysorbate, 6 to 12 parts by weight of polyvinyl alcohol, 2 to 15 parts by weight of polyvinyl pyrrolidone, 0.3 to 6 parts by weight of a color, 1 to 30 parts by weight of dihydroxy acetone, 0.1 to 0.5 part by weight of phenoxy ethanol, 0.1 to 0.5 part by weight of citric acid, and 5 to 30 parts by weight of ethyl alcohol.

2. The peel-off type eyebrow coloring composition as claimed in claim 1, wherein the composition further comprises 10 to 20 parts by weight of butylene glycol.

3. The peel-off type eyebrow coloring composition as claimed in claim 1, wherein the composition further comprises 1 to 5 parts by weight of 1,2-hexanediol.

4. The peel-off type eyebrow coloring composition as claimed in claim 1, wherein the composition further comprises a fragrance.

5. A method for manufacturing a peel-off type eyebrow coloring composition, comprising:
(a) dissolving 0.01 to 0.05 part by weight of disodium EDTA and 0.5 to 2 parts by weight of polysorbate in 15 to 30 parts by weight of water at 75 to 85 °C to prepare a first solution;
(b) gradually adding 6 to 12 parts by weight of polyvinyl alcohol to the first solution under agitation and performing a complete dissolution to prepare a second solution;
(c) gradually adding 2 to 15 parts by weight of polyvinyl pyrrolidone to the second solution under agitation and completing a dispersion to prepare a third solution;
(d) adding 0.3 to 6 parts by weight of a color and 1 to 30 parts by weight of dihydroxy acetone to the third solution under agitation and completing a dispersion to prepare a fourth solution;
(e) adding 0.1 to 0.5 part by weight of phenoxy ethanol and 0.1 to 0.5 part by weight of citric acid to the fourth solution under agitation and completing a dispersion to prepare a fifth solution; and
(f) adding 5 to 30 parts by weight of ethyl alcohol to the fifth solution under agitation and completing a dispersion to prepare a sixth solution.

## Patentansprüche

1. Abziehbaren Augenbrauenfärbezusammensetzung, umfassend 15 bis 30 Gew.-Teilen Wasser, 0,01 bis 0,05 Gew.-Teilen Dinatrium-EDTA, 0,5 bis 2 Gew.-Teilen Polysorbat, 6 bis 12 Gew.-Teilen Polyvinylalkohol, 2 bis 15 Gew.-Teilen Polyvinylpyrrolidon, 0.3 bis 6 Gew.-Teilen einer Farbe, 1 bis 30 Gew.-Teilen Dihydroxyaceton, 0,1 bis 0,5 Gew.-Teilen Phenoxyethanol, 0,1 bis 0,5 Gew.-Teilen Zitronensäure und 5 bis 30 Gew.-Teilen Ethylalkohol.

2. Abziehbaren Augenbrauenfärbezusammensetzung nach Anspruch 1, wobei die Zusammensetzung ferner 10 bis 20 Gew.-Teilen Butylenglykol umfasst.

3. Abziehbaren Augenbrauenfärbezusammensetzung nach Anspruch 1, wobei die Zusammensetzung ferner 1 bis 5 Gew.-Teilen 1,2-Hexandiol umfasst.

4. Abziehbaren Augenbrauenfärbezusammensetzung nach Anspruch 1, wobei die Zusammensetzung ferner einen Duft umfasst.

5. Verfahren zur Herstellung einer abziehbaren Augenbrauenfärbezusammensetzung, umfassend:
(a) Lösen von 0,01 bis 0,05 Gew.-Teilen Dinatrium-EDTA und 0,5 bis 2 Gew.-Teilen Polysorbat in 15 bis 30 Gew.-Teilen Wasser bei 75 bis 85 °C zur Herstellung einer ersten Lösung;
(b) allmähliche Zugabe von 6 bis 12 Gew.-Teilen Polyvinylalkohol zu der ersten Lösung unter Rühren und Durchführung einer vollständigen Auflösung zur Herstellung einer zweiten Lösung;
(c) allmähliche Zugabe von 2 bis 15 Gew.-Teilen Polyvinylpyrrolidon zur zweiten Lösung unter Rühren und Vervollständigung einer Dispersion zur Herstellung einer dritten Lösung;
(d) Zugabe von 0,3 bis 6 Gew.-Teilen einer Farbe und 1 bis 30 Gew.-Teilen Dihydroxyaceton zur dritten Lösung unter Rühren und Vervollständigung einer Dispersion zur Herstellung einer vierten Lösung;
(e) Zugabe von 0,1 bis 0,5 Gew.-Teilen Phenoxyethanol und 0,1 bis 0,5 Gew.-Teilen Zitronensäure zur vierten Lösung unter Rühren und Vervollständigung einer Dispersion zur Herstellung einer fünften Lösung; und
(f) Zugabe von 5 bis 30 Gew.-Teilen Ethylalkohol zur fünften Lösung unter Rühren und Vervollständigung einer Dispersion zur Herstellung einer sechsten Lösung.

## Revendications

1. Composition colorante pour sourcils du type pelable comprenant 15 à 30 parties en poids d'eau, 0,01 à 0,05 partie en poids d'EDTA disodique, 0,5 à 2 parties en poids de polysorbate, 6 à 12 parties en poids d'alcool polyvinylique, 2 à 15 parties en poids de polyvinylpyrrolidone, 0.3 à 6 parties en poids d'une couleur, 1 à 30 parties en poids de dihydroxyacétone, 0,1 à 0,5 partie en poids de phénoxyéthanol, 0,1 à 0,5 partie en poids d'acide citrique et 5 à 30 parties en poids d'alcool éthylique.

2. Composition colorante pour sourcils du type pelable selon la revendication 1, dans laquelle la composition comprend en outre 10 à 20 parties en poids de butylèneglycol.

3. Composition colorante pour sourcils du type pelable selon la revendication 1, dans laquelle la composition comprend en outre 1 à 5 parties en poids de 1,2-hexanediol.

4. Composition colorante pour sourcils du type pelable selon la revendication 1, dans laquelle la composition comprend en outre un parfum.

5. Procédé de fabrication d'une composition colorante pour sourcils du type pelable, comprenant :
(a) la dissolution de 0,01 à 0,05 partie en poids d'EDTA disodique et de 0,5 à 2 parties en poids de polysorbate dans 15 à 30 parties en poids d'eau à 75 à 85 °C pour préparer une première solution ;
(b) ajouter progressivement 6 à 12 parties en poids d'alcool polyvinylique à la première solution sous agitation et effectuer une dissolution complète pour préparer une seconde solution ;
(c) ajouter progressivement 2 à 15 parties en poids de polyvinylpyrrolidone à la deuxième solution sous agitation et compléter une dispersion pour préparer une troisième solution ;
(d) ajouter 0,3 à 6 parties en poids d'une couleur et 1 à 30 parties en poids de dihydroxyacétone à la troisième solution sous agitation et compléter une dispersion pour préparer une quatrième solution ;
(e) ajouter 0,1 à 0,5 partie en poids de phénoxyéthanol et 0,1 à 0,5 partie en poids d'acide citrique à la quatrième solution sous agitation et compléter une dispersion pour préparer une cinquième solution ; et
(f) ajouter 5 à 30 parties en poids d'alcool éthylique à la cinquième solution sous agitation et compléter une dispersion pour préparer une sixième solution.
